(19) Europäisches Patentamt
European Patent Office
Office européen des brevets

(11) **EP 2 567 618 B1**

(12) **FASCICULE DE BREVET EUROPEEN**

(45) Date de publication et mention
de la délivrance du brevet:
**03.05.2017  Bulletin 2017/18**

(21) Numéro de dépôt: **12195560.3**

(22) Date de dépôt: **16.12.2008**

(51) Int Cl.:
*A01N 47/02* *(2006.01)*  *A01N 25/02* *(2006.01)*
*A01P 7/04* *(2006.01)*  *A61K 31/415* *(2006.01)*
*A61K 47/34* *(2017.01)*  *A61P 33/14* *(2006.01)*
*A61K 9/00* *(2006.01)*

(54) **Composition pharmaceutique contenant un dérivé de N-phénylpyrazole, utilisation pour la préparation d'un médicament vétérinaire topique pour lutter contre les puces**

Pharmazeutische Zusammensetzung, die ein N-Phenylpyrazol-Derivat umfasst, und deren Verwendung für die Herstellung eines lokalen Veterinärmedikaments zur Bekämpfung von Flöhen

Pharmaceutical composition containing an N-phenylpyrazole derivative, use for the preparation of a topical veterinary drug for flea control

(84) Etats contractants désignés:
**AT BE BG CH CY CZ DE DK EE ES FI FR GB GR HR HU IE IS IT LI LT LU LV MC MT NL NO PL PT RO SE SI SK TR**

(43) Date de publication de la demande:
**13.03.2013  Bulletin 2013/11**

(62) Numéro(s) de document de la (des) demande(s) initiale(s) en application de l'article 76 CBE:
**08875636.6 / 2 375 905**

(73) Titulaire: **VIRBAC**
**06516 Carros (FR)**

(72) Inventeur: **Derrieu, Guy**
**06800 Cagnes sur Mer (FR)**

(74) Mandataire: **Gevers & Orès**
**41 avenue de Friedland**
**75008 Paris (FR)**

(56) Documents cités:
**WO-A-97/12521**

- **MERIAL ANIMAL HEALTH LIMITED: "Summary of product and characteristics. Frontline Spot On Cat", INTERNET ARTICLE , 2007, XP007905533, Extrait de l'Internet: URL:www.vmd.gov.uk/espcsite/Documents/138 1 28.DOC [extrait le 2008-08-26]**

EP 2 567 618 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente Invention se rapporte à une composition pharmaceutique liquide renfermant, à titre de principe actif, du fipronil, et à titre de véhicule, de l'alcool benzylique et un solvant organique convenablement sélectionné, à son procédé de préparation ainsi qu'à l'utilisation de ce véhicule pour l'amélioration de la rémanence de la composition.

Les animaux de compagnie sont souvent infestés par un ou plusieurs parasites se nourrissant de sang tels que les puces du chien ou du chat, les tiques, ou encore les gales.

Les puces sont des insectes dépourvus d'ailes, au corps comprimé latéralement et aux pattes très développées, adaptées au saut. Ce sont des ectoparasites, suceurs de sang de mammifères ou d'oiseaux. Les quelques 2000 espèces répertoriées appartiennent à l'ordre des siphonaptères. Deux espèces de puces sont communément rencontrées en Europe ; il s'agit de la puce du chat (*Ctenocephalides felis*) et de la puce du chien (*Ctenocephalides canis*) qui vivent dans la fourrure des animaux. La puce du chat, la plus fréquente, est capable de se reproduire à la fois sur le chat et le chien. Elle peut aussi s'attaquer à l'homme et aux autres animaux de compagnie, cependant le chat est le principal responsable d'infestation lorsque chats et chiens vivent dans le même environnement.

Les puces ont un cycle de vie complexe avec 4 stades distincts : oeuf, larve, nymphe et adulte. Elles s'accouplent dans les premières 8 à 48 heures suivant l'acquisition par l'hôte, après leur premier repas sanguin. Les femelles commencent ainsi à pondre 24 à 48 heures après ce premier repas sanguin. La puce adulte pond généralement sur l'animal. Les oeufs pondus sur l'animal n'y restent d'ailleurs pas et tombent sur le sol. En conditions optimales, la femelle peut pondre plus de 25 oeufs par jour. Elle en pondra plusieurs centaines durant toute sa vie. Après quelques jours, naît une larve vermiforme, blanche et poilue, d'environ 1,5 mm de long. La larve se nourrit de débris organiques, de dépouilles larvaires et du sang sec déféqué par les adultes. L'état larvaire dure de 1 à 3 semaines, si les conditions sont favorables (18° à 27°C et 70% d'humidité relative). La larve tisse ensuite un cocon et se nymphose. Normalement, la nymphe évolue en 1 à 2 semaines mais le passage à l'état adulte peut se prolonger jusqu'à 1 an, si les conditions sont défavorables. La puce adulte (petite et noire) émerge du cocon quand elle décèle vibrations, chaleur, concentration plus élevée en gaz carbonique, ce qui se produit lors du passage d'un chat, d'un chien... ou d'un homme ! Elle saute alors sur la victime, se nourrit aussitôt de sang et grossit rapidement en prenant une couleur plus claire d'un brun rougeâtre. La puce adulte vit de 6 à 12 mois. Sans nourriture, elle peut survivre jusqu'à 2 mois.

Les piqûres de puces provoquent des démangeaisons, chez l'animal comme chez l'homme. La salive de la puce (sécrétée à chaque piqûre) peut aussi, selon les individus, entraîner des réactions allergiques, immédiates ou retardées. Ces réactions se traduisent par diverses lésions cutanées et démangeaisons. On distingue deux types de dermatoses liées aux puces, à savoir la pulicose et la dermatite par allergie aux piqûres de puces. Si dans les deux cas la dermatose résulte d'une infestation plus ou moins importante par des puces, ce n'est que dans la seconde qu'un phénomène allergique est associé. La dermatite par allergie aux piqûres de puces (DAPP) est la cause la plus fréquente de prurit chez le chien. En France, chez le chien adulte, elle représente ainsi près de la moitié des dermatoses prurigineuses. Près de 80 % des chiens qui présentent une DAPP ont également une dermatite atopique. Réciproquement, deux chiens atopiques sur trois présentent une DAPP. Il est donc probable que les chiens atopiques soient prédisposés au développement d'une allergie aux piqûres de puces et que l'infestation par celles-ci soit un facteur déclenchant d'une dermatite atopique. Cela justifie de la nécessité d'un contrôle antipuce très poussé chez les chiens atopiques ou appartenant à des races à risque. De plus, la DAPP est probablement la principale cause de réapparition du prurit chez les chiens atopiques désensibilisés.

Les puces du genre *Ctenocephalides* sont par ailleurs des hôtes intermédiaires de *Dipylidium caninum*, qui est un vers parasite de l'intestin grêle du chien et du chat. Le carnivore s'infeste en avalant les puces parasitées. Cette infestation peut provoquer un prurit anal, un engorgement des sacs anaux, ainsi qu'une dermatite de la région périnéale. C'est pourquoi, il est parfois conseillé de vermifuger régulièrement les animaux en complément de la lutte contre les puces.

De la même manière, les tiques (*Rhipicephalus sp.*, *Ixodes sp.*, *Dermacentor sp.*, *Amblyomma sp.*, ...) peuvent aussi causer un stress à l'animal et nuire à sa santé. Elles peuvent aussi nuire à l'homme. Mais le plus grave problème des tiques est qu'elles sont un vecteur d'agents pathogènes pouvant concerner l'animal autant que l'homme. Parmi les maladies majeures devant être évitées, on peut citer les Borellioses (maladie de Lyme à *Borellia burgdorferi*), les Babésioses (piroplasmoses à *Babesia sp.*), et les Rickettsioses. Les tiques peuvent aussi libérer des toxines aux propriétés paralysantes et inflammatoires, et parfois mortelles.

La galle (*Demodex sp.*, *Sarcoptes sp.*, *Otodectes sp.*, ...) est particulièrement difficile à combattre car il existe très peu de matières actives efficaces. Elle requiert des traitements fréquents.

Les infestations par ces différents parasites, et tout particulièrement par les puces, représentent donc un important problème sanitaire pour les animaux qui sont infestés et imposent de pouvoir disposer de traitements adaptés. Il convient en particulier que le traitement ait non seulement une efficacité immédiate (rapidité d'action) mais également une efficacité prolongée dans le temps (rémanence) afin d'éviter, d'une part, les traitements répétés et, d'autre part, tout risque d'infestation et/ou de réinfestation pendant une période prolongée. La puce, en particulier, doit être éliminée avant qu'elle ne se reproduise et commence à pondre.

Il existe beaucoup substances insecticides plus ou moins actives et plus ou moins coûteuses. Des phénomènes de résistance apparaissent liés à leur utilisation, c'est notamment le cas lors de l'utilisation des carbamates, des composés organophosphorés et des pyréthroïdes.

Par ailleurs, les demandes de brevets EP 0 295 117 et EP 0 352 944 décrivent une grande famille de N-phénylpyrazoles ayant un très large spectre d'activité, y compris des activités antiparasitaires.

Bien qu'efficaces, les dérivés de N-phénylpyrazoles, et en particulier le 5-amino-1-[2,6-dichloro-4-(trifluorométhyl)phényl]-4-(trifluorométhylsulfinyl)-1H-pyrazole-3-carbonitrile (fipronil), sont parfois difficiles à formuler car ils ne présentent pas toujours une solubilité suffisante dans les excipients classiquement utilisés pour la préparation de compositions antiparasitaires liquides prêtes à l'emploi.

En effet, les produits actifs contre les parasites suceurs de sang, et en particulier contre les puces, peuvent se présenter notamment sous la forme de compositions liquides (pipettes) ou solutions cutanées pour dépôt ou encore "Spot-On", à appliquer très facilement, en une seule fois, de façon topique directement sur la peau de l'animal, généralement entre les omoplates.

Cependant, dans ce type de composition, le fipronil est souvent difficile à formuler et peut conduire à des phénomènes de cristallisation. Afin de surmonter ce problème, il a déjà été proposé, notamment dans la demande de brevet EP 0 881 881, de formuler les dérivés de N-phénylpyrazoles en milieu solvant en présence d'un inhibiteur de cristallisation et d'un alcool en $C_1$-$C_4$. Le produit Frontline® Spot-On Chat et Chien, vendu en France par la société Merial SAS s'inscrit dans cette technologie.

De telles compositions, si elles sont bien adaptées pour éviter les problèmes de cristallisation de ces principes actifs particuliers, ne donnent cependant pas toujours entière satisfaction d'un point de vue de la durée de protection qu'elles confèrent à l'animal. Dans le cas du produit Frontline® Spot-On Chat et Chien notamment, la durée de protection contre les nouvelles infestations par les puces annoncée par le fabricant est limitée à 4 semaines chez le chat et à 2 mois chez le chien. Cependant, des essais d'efficacité anti-parasitaire conduits selon les standards actuels ne permettent pas de reproduire les résultats d'efficacité prolongée et le produit ne dispose donc pas toujours d'une rémanence totalement satisfaisante.

C'est donc afin de remédier à l'ensemble des problèmes rencontrés avec les produits antiparasitaires actuellement disponibles sur le marché et de pourvoir à un produit permettant de prévenir et de traiter efficacement les infestations par les puces chez les animaux domestiques, aussi bien chez le chat que chez le chien, que la Demanderesse a mis au point ce qui fait l'objet de l'Invention. Elles s'est en particulier donné pour but de pourvoir à un produit pour la prévention et le traitement des infestations par les puces chez les animaux domestiques qui soit facile à formuler, facile à administrer, tout en ayant une action rapide et plus rémanente que les produits actuellement disponibles sur le marché.

Un autre objectif de l'invention est de fournir de telles compositions facilement utilisables quelles que soient l'espèce animale, la taille de l'animal ou la nature de son pelage.

Un autre objectif de l'invention est de disposer de compositions efficaces ne nécessitant pas de mouiller tout l'animal.

Ces objectifs sont atteints par la composition pharmaceutique antiparasitaire qui fait l'objet de la présente invention.

De manière inattendue, la composition pharmaceutique antiparasitaire de l'invention fournit une activité efficace et prolongée dans le traitement et la protection des animaux domestiques sous forme de solution prête à l'emploi d'utilisation aisée.

Ainsi, la présente Invention a pour objet une composition pharmaceutique liquide, conditionnée en pipettes monodose et caractérisée par le fait qu'elle renferme :

- à titre de principe actif, le 5-amino-1-[2,6-dichloro-4-(trifluorométhyl)phényl]-4-(trifluorométhylsulfinyl)-1 H-pyrazole-3-carbonitrile (fipronil),
- au moins 5 % (poids/volume) d'alcool benzylique, et
- au moins 50 % (poids/volume) d'un solvant organique choisi parmi le monométhyléther du propylèneglycol, le n-butyléther du dipropylèneglycol, le monométhyléther de l'éthylèneglycol, le monoéthyléther de l'éthylèneglycol, le monoéthyléther du diéthylène glycol, le propylène glycol, et leurs mélanges,

étant entendu que ladite composition est exempte d'alcool en $C_1$-$C_4$.

Selon l'Invention, ladite composition pharmaceutique est en particulier destinée à être administrée chez le chat ou chez le chien.

Au sein de la composition pharmaceutique conforme à l'Invention, le fipronil représente de préférence de 1 à 20 % (poids/volume), et encore plus préférentiellement de 5 à 15 % (poids/volume). Il doit être bien entendu, toutefois, que ces quantités sont données à titre indicatif et qu'elles peuvent être modulées selon les besoins de la formulation notamment eu égard aux doses efficaces en fonction de l'animal à traiter et de son poids.

Selon une forme de réalisation préférée de l'invention, l'alcool benzylique représente de 5 à 40 % (poids/volume), et encore plus préférentiellement de 25 à 35 % (poids/volume).

Le ou les solvants organiques mentionnés ci-dessus sont utilisés dans les quantités nécessaires pour ajuster la composition pharmaceutique au volume final requis. Le pourcentage poids/volume du solvant organique ou du mélange de solvants organiques dépend de la nature (donc de la densité) du solvant ou du mélange de solvants utilisés.

Parmi lesdits solvants organiques, le monoéthyl du diéthylèneglycol (EDG) est tout particulièrement préféré.

La composition pharmaceutique utilisée conformément à l'Invention peut en outre renfermer un ou plusieurs excipients pouvant par exemple être choisis parmi les agents tensioactifs, les agents épaississants, les colorants, les parfums, les antioxydants parmi lesquels on peut citer à titre d'exemple non limitatif le butylhydroxyanisole, le butylhydroxytoluène, le gallate de propyle, le palmitate d'ascorbyle, les extraits de romarin et leurs mélanges.

Lorsqu'il(s) est (sont) présent(s), le ou les agents antioxydants représentent de préférence de 0,005 à 2 % environ (poids/volume), et encore plus préférentiellement de 0,01 à 0,1 % environ (poids/volume).

Outre le fipronil, la composition pharmaceutique peut comprendre également un ou plusieurs principes actifs antiparasitaires additionnels. A titre de principe actif antiparasitaire additionnel, on peut notamment mentionner les acaricides tels que l'amitraz ou le cymiazole, les inhibiteurs du développement des puces et des tiques, souvent appelés "IGR" pour "Insect Growth Regulators" en anglais, tels que le pyriproxyfène et l'éthoxazole, les endoparasiticides tels que les avermectines et leurs dérivés comme par exemple l'ivermectine, l'abamectine, la doramectine et la moxydectine, les milbémycines, ainsi que les composés actifs contre les phlébotomes et les ectoparasites des animaux domestiques.

De telles combinaisons d'actifs peuvent être utiles en vue d'élargir le spectre d'action de la composition conforme à la présente invention.

La composition pharmaceutique conforme à l'Invention peut facilement être préparée par simple dilution du fipronil et éventuellement du ou des principes actifs antiparasitaires additionnels dans l'alcool benzylique et le ou les solvants organiques utilisés.

Après sa préparation, la composition pharmaceutique est de préférence conditionnée en pipettes monodoses.

Un autre objet de la présente demande est l'utilisation d'une composition pharmaceutique liquide telle que décrite précédemment pour la préparation d'un médicament vétérinaire antiparasitaire à application topique pour la prévention (protection) et/ou le traitement des infestations par les puces chez les animaux domestiques, en particulier chez les chiens ou les chats.

Selon cette utilisation, ledit médicament est destiné à être appliqué par application directe sur la peau de l'animal, au niveau des omoplates ou sur une ligne dorsale partant de la base de la queue et remontant jusqu'au cou.

La quantité de médicament à administrer peut varier de 0,3 à 1,5 ml environ, de préférence de 0,5 ml environ chez le chat et de 0,3 à 6,0 ml environ chez le chien, en fonction du poids de l'animal considéré et de la posologie.

Le volume à appliquer selon l'invention doit correspondre de préférence à une dose unitaire de fipronil allant de 0,3 à 60 mg par kg de poids corporel, et encore plus préférentiellement de 5 à 15 mg par kg de poids corporel.

Ainsi selon une forme de réalisation préférée de l'invention, ledit médicament est destiné à administrer une dose unitaire de fipronil allant de 0,3 à 60 mg par kg de poids corporel, et encore plus préférentiellement de 5 à 15 mg par kg de poids corporel.

Outre les dispositions qui précèdent, l'Invention comprend encore d'autres dispositions qui ressortiront de la description qui va suivre, qui se réfère à un exemple de préparation d'une composition pharmaceutique conforme à l'invention, ainsi qu'à des exemples mettant en évidence l'efficacité de ladite composition vis-à-vis du traitement des puces chez le chien et chez le chat.

### EXEMPLE 1 : COMPOSITION ANTIPUCES

On a préparé la composition antipuce suivante, par simple dissolution du fipronil dans le mélange des autres constituants de la composition :

| | |
|---|---|
| - Fipronil | 10,0 g |
| - Alcool benzylique | 30,0 g |
| - Butylhydroxyanisole | 0,02 g |
| - Butylhydroxytoluène | 0,01 g |
| - Monoéthyléther du diéthylène glycol q.s.p. | 100 ml |

Après préparation, cette composition peut être directement conditionnée en pipettes monodoses.

## EXEMPLE 2 : ÉTUDE DE L'EFFICACITÉ IMMÉDIATE ET PROLONGÉE D'UNE COMPOSITION CONFORME A L'INVENTION CONTRE LES PUCES CHEZ LE CHIEN

Dans cet exemple, on a réalisé une étude destinée à déterminer et à comparer les efficacités immédiates et prolongées de deux compositions topiques à base de fipronil :

- une composition A conforme à l'invention et telle que décrite ci-dessus à l'exemple 1 ;
- le produit commercialisé sous la dénomination Frontline® Spot-on dog par la société Mérial.

### 1) Matériel et Méthodes

#### a) Type d'étude

Il s'agit d'une étude d'efficacité contrôlée en aveugle, randomisée, réalisée en parallèle sur 3 groupes de huit chiens.

#### b) Animaux utilisés et conditions de maintenance

Les chiens utilisés dans cette étude étaient des chiens domestiques adultes, males ou femelles, âgés de plus de 4 mois, de races mélangées, mais principalement de race européenne à poils courts, pesant entre 2 et 20 kg. Avant le début de l'étude, il a été vérifié que tous les chiens étaient en bonne santé, qu'ils n'étaient pas infestés de puces et que les femelles n'étaient pas enceintes. Tous les chiens ont été vermifugés et acclimatés aux conditions de vie pendant au moins 7 jours avant le démarrage de l'étude.

Pendant la période d'acclimatation et toute la durée de l'étude, les chiens ont été gardés à l'intérieur dans une pièce climatisée, chaque chien étant confiné dans un enclos individuel de dimensions 1,9 m x 2,97 m sans litière et sans contact possible entre les différents chiens engagés dans l'étude. Le numéro d'identification, le numéro de groupe et le type de composition administrée a été noté à l'extérieur de chaque enclos. La température de la pièce a été maintenue à environ 20°C $\pm$ 4 °C. Les chiens ont été soumis à une alternance de 12 heures de lumière et de 12 heures d'obscurité.

Les animaux ont été nourris une fois par jour avec des croquettes pour chien du commerce vendues sous la dénomination commerciale Eukanuba ® par la société Iams, une division de Foodcorp., selon les recommandations du fabricant, et ils ont eu de l'eau potable fraîche à volonté.

#### c) Compositions testées

La composition A conforme à l'invention a été comparée au produit Frontline® Spot-on dog contenant 10 % (g/100 ml) de fipronil et un mélange d'excipients. Il a été utilisé tel que fourni par le fabriquant.

#### d) Traitements

Groupe 1 : Traitement avec la composition A à raison de 0,67 ml par chien pour les chiens pesant entre 2 et 10 kg, et à raison de 1,34 ml par chien pour les chiens pesant entre plus de 10 kg et 20 kg,

Groupe 2 : Traitement avec le produit Frontline® Spot-on dog à raison de 0,67 ml chien pour les chiens pesant entre 2 et 10 kg, et à raison de 1,34 ml par chien pour les chiens pesant entre plus de 10 kg et 20 kg,

Groupe 3 : Contrôle négatif : pas de traitement,

Le traitement a été appliqué de façon topique, entre les omoplates des chiens, en une seule fois au commencement de l'étude (J = 0).

#### e) Infestations par les puces / Mesure de l'efficacité des traitements

6 jours avant le commencement de l'étude (J = -6), tous les chiens ont été infestés par environ 100 puces de laboratoire, souche *Ctenocephalis felis,* de sexe male ou femelle. Les puces ont ensuite été comptées 4 jours avant le début du traitement (J = -4). Pour ce faire, toutes les puces présentes sur un animal sont récoltées par peignage du chien puis comptées après peignage. Le nombre de puces est ainsi déterminé. Après comptage, les puces sont détruites et un nouveau lot d'environ 100 puces est remis sur l'animal le jour précédent l'administration du traitement (J = - 1).

Il a ensuite été procédé au comptage du nombre de puces encore vivantes 2 jours après l'administration de la composition testée (J = 2).

Les chiens ont à nouveau été infestés par une quantité connue de puces (environ 100) 7 jours (J = 7), 14 jours (J = 14), 21 jours (J = 21), 28 jours (J = 28), 35 jours (J = 35), 42 jours (J = 42), 49 jours (J = 49), 56 jours (J = 56), 63 jours (J = 63), 70 jours (J = 70), 77 jours (J = 77), 84 jours (J = 84) et 91 jours (J = 91), après l'administration du traitement.

Un comptage des puces encore vivantes a alors été effectué 48 heures après chacune de ces nouvelles infestations (J = 9 ; J = 16 ; J = 23 ; J = 30 ; J = 37; J=44; J=51; J=58; J=65; J=72; J=79; J=86 et J=93).

A chaque comptage, l'efficacité du traitement a été calculée selon l'équation suivante : % d'efficacité = 100 x $(NP_vC - NP_vT) / NP_vC$

dans laquelle :

- $NP_vC$ est la moyenne géométrique du nombre de puces vivantes comptées sur les chiens du groupe 3 (contrôle) ;
- $NP_vT$ est la moyenne géométrique du nombre de puces vivantes comptées sur les chiens d'un groupe ayant reçu un traitement (Groupe 1 ou 2).

Un traitement est dit efficace si le pourcentage d'efficacité est supérieur ou égal à 95%.

## 2) Résultats

Les résultats moyens obtenus sont reportés dans le tableau I ci après :

### TABLEAU I

| Jours | GROUPE 1 (Composition A) | GROUPE 2 (Frontline ® Spot-on dog) |
|---|---|---|
| J = 2 | 99.7 | 100.0 |
| J = 9 | 100.0 | 100.0 |
| J = 16 | 99.8 | 100.0 |
| J = 23 | 100.0 | 100.0 |
| J = 30 | 100.0 | 100.0 |
| J = 37 | 100.0 | 100.0 |
| J = 44 | 99.9 | 100.0 |
| J = 51 | 100.0 | 100.0 |
| J = 58 | 100.0 | 99.6 |
| J = 65 | 99.9 | 99.3 |
| J = 72 | 99.8 | 98.2 |
| J = 79 | 98.9 | 96.4 |
| **J = 86** | **99.2** | **85.5** |
| **J = 93** | **97.1** | **75.4** |

Ces résultats montrent que :

- la composition A conforme à la présente invention reste efficace, (détermination au bout de 48 heures après l'infestation) pendant 13 semaines (J = 93) contre les infestations par les puces chez le chien ;
- le produit Frontline ® Spot-on dog ne reste efficace (détermination au bout de 48 heures après l'infestation) que pendant 11 semaines (J = 79) contre les infestations par les puces chez le chien.

L'efficacité et la meilleure rémanence de la composition A conforme à la présente Invention sont ainsi clairement démontrées.

**EXEMPLE 3 : ÉTUDE DE L'EFFICACITÉ D'UNE COMPOSITION CONFORME A L'INVENTION CONTRE LES PUCES CHEZ LE CHIEN**

Dans cet exemple, on a réalisé une étude destinée à déterminer et à comparer l'efficacité de deux compositions topiques à base de fipronil :

- une composition A conforme à l'invention et telle que décrite ci-dessus à l'exemple 1 ;
- le produit commercialisé sous la dénomination Frontline® Spot-on dog par la société Mérial.

**1) Matériel et Méthodes**

a) Type d'étude

Il s'agit d'une étude d'efficacité contrôlée en aveugle, randomisée, réalisée en parallèle sur 3 groupes de six chiens.

b) Animaux utilisés et conditions de maintenance

Les chiens utilisés dans cette étude étaient des chiens bâtards appartenant à l'espèce *Canis familiaris*, males ou femelles, âgés de plus de 6 mois, pesant entre 6 kg et 25 kg. Avant le début de l'étude, il a été vérifié que tous les chiens étaient en bonne santé et qu'ils n'étaient pas infestés de puces. Tous les chiens ont été vermifugés et acclimatés aux conditions de vie pendant au moins 7 jours avant le démarrage de l'étude.

Il a également été vérifié que les chiens n'avaient reçu aucun traitement topique contre les puces au cours de 12 semaines précédent le début de l'étude.

Pendant la période d'acclimatation et toute la durée de l'étude, les chiens ont été gardés à l'intérieur dans une pièce climatisée, chaque chien étant confiné dans un enclos individuel de dimensions 1,9 m x 2,97 m sans litière et sans contact possible entre les différents chiens engagés dans l'étude. Le numéro d'identification, le numéro de groupe et le type de composition administrée a été noté à l'extérieur de chaque enclos. La température de la pièce a été maintenue à environ 20°C $\pm$ 4 °C. Les chiens ont été soumis à une alternance de 12 heures de lumière et de 12 heures d'obscurité.

Les animaux ont été nourris une fois par jour avec des croquettes pour chien du commerce vendues sous la dénomination commerciale Ultradog Superwoof par la société Nola, une division de Foodcorp., selon les recommandations du fabricant, et ils ont eu de l'eau potable fraîche à volonté.

c) Compositions testées

La composition A conforme à l'invention a été comparée au produit Frontline® Spot-on dog contenant 10 % (g/100 ml) de fipronil et un mélange d'excipients. Il a été utilisé tel que fourni par le fabriquant.

d) Traitements

Groupe 1 : Traitement avec la composition A à raison de 0,067 ml par kg de poids corporel,
Groupe 2 : Traitement avec le produit Frontline® Spot-on dog à raison de 0,067 ml par kg de poids corporel,
Groupe 3 : Contrôle négatif : pas de traitement,

Le traitement a été appliqué de façon topique, entre les omoplates des chiens, en une seule fois au commencement de l'étude (J = 0).

e) Infestations par les puces / Mesure de l'efficacité des traitements

6 jours avant le commencement de l'étude (J = -6), tous les chiens ont été infestés par environ 100 puces de laboratoire, souche *Ctenocephalis felis*, de sexe male ou femelle. Les puces ont ensuite été comptées 5 jours avant le début du traitement (J = -5). Pour ce faire, toutes les puces présentes sur un animal sont récoltées par peignage du chien puis comptées après peignage. Le nombre de puces est ainsi déterminé. Après comptage, les puces sont détruites et un nouveau lot d'environ 100 puces est remis sur l'animal le jour précédent l'administration du traitement (J = - 2).

Il a ensuite été procédé au comptage du nombre de puces encore vivantes 1 jour après l'administration de la composition (J = 1).

Les chiens ont à nouveau été infestés par une quantité connue de puces (environ 100) 7 jours (J = 7), 14 jours (J = 14), 21 jours (J = 21), 35 jours (J = 35), 42 jours (J = 42), 49 jours (J = 49) et 56 jours (J = 56) après l'administration du traitement.

Un comptage des puces encore vivantes a alors été effectué 24 heures après chacune de ces nouvelles infestations (J = 8 ; J = 15; J = 22 ; J = 36 ; J = 43; J = 50 et J = 57).

A chaque comptage, l'efficacité du traitement a été calculée selon l'équation suivante : % d'efficacité = 100 x $(NP_vC - NP_vT) / NP_vC$

dans laquelle :

- $NP_vC$ est la moyenne géométrique du nombre de puces vivantes comptées sur les chiens du groupe 3 (contrôle) ;
- $NP_vT$ est la moyenne géométrique du nombre de puces vivantes comptées sur les chiens d'un groupe ayant reçu un traitement (Groupe 1 ou 2).

Un traitement est dit efficace si le pourcentage d'efficacité est supérieur ou égal à 95%.

## 2) Résultats

Les résultats moyens obtenus sont reportés dans le tableau II ci-après :

**TABLEAU II**

| Jours | GROUPE 1 (Composition A) | GROUPE 2 (Frontline ® Spot On Dog) |
|-------|--------------------------|-------------------------------------|
| J = 1 | 92.2 | 84.4 |
| J = 8 | 99.5 | 99.8 |
| J = 15 | 100.0 | 99.8 |
| J = 22 | 99.6 | 100.0 |
| J = 29 | 100.0 | 98.7 |
| J = 36 | 100.0 | 96.9 |
| J = 43 | 99.2 | 96.8 |
| J = 50 | 97.0 | 93.7 |
| J = 57 | 89.4 | 74.8 |

Ces résultats montrent que :

- la composition A agit plus rapidement que le produit Frontline® Spot-on dog (comparaison des % d'efficacité à J = 1) ;
- la composition A conforme à la présente invention reste efficace (détermination au bout de 24 heures après l'infestation) pendant 7 semaines (J = 50) contre les infestations par les puces chez le chien ;
- le produit Frontline ® Spot-on dog ne reste efficace (détermination au bout de 24 heures après l'infestation) que pendant 6 semaines (J = 43) contre les infestations par les puces chez le chien.

La rapidité d'action et la meilleure rémanence de la composition A conforme à la présente Invention sont ainsi clairement démontrées.

## EXEMPLE 4 : ÉTUDE DE L'EFFICACITÉ D'UNE COMPOSITION CONFORME A L'INVENTION CONTRE LES PUCES CHEZ LE CHAT

Dans cet exemple, on a réalisé une étude destinée à déterminer et à comparer l'efficacité de deux compositions topiques à base de fipronil :

- une composition A conforme à l'invention et telle que décrite ci-dessus à l'exemple 1 ;
- le produit commercialisé sous la dénomination Frontline® Spot-on cat par la société Mérial.

## 1) Matériel et Méthodes

### a) Type d'étude

Il s'agit d'une étude d'efficacité contrôlée en aveugle, randomisée, réalisée en parallèle sur 3 groupes de six chats.

Cette étude a été conduite selon les Bonnes Pratiques de Laboratoire pour l'évaluation des produits vétérinaires (ENV / MC / CHEM / (98)17 ; arrêté du 28 janvier 2005, publié au JO du 20 février 2005) et selon les recommandations des Directives du Comité sur les produits médicinaux vétérinaires ("Committee for Veterinary Medicinal Products" (CVMP) : "*Guidelines for the testing and evaluation of the efficacy of anti parasitic substances for the treatment and prevention of tick and flea infestations in dogs and cats*", EMEA/CVMP/005/2000-Rev.2).

### b) Animaux utilisés et conditions de maintenance

Les chats utilisés dans cette étude étaient des chats domestiques européens adultes, males ou femelles, âgés de 7 mois à 2 ans, pesant en moyenne 6,7 ± 0,1 kg. Tous les chats acclimatés aux conditions de vie pendant au moins 9 jours avant le démarrage de l'étude.

Pendant la période d'acclimatation et toute la durée de l'étude, les chats ont été gardés à l'intérieur dans une pièce climatisée, chaque chat étant confiné dans des cages individuelles. Le numéro d'identification, le numéro de groupe et le type de composition administrée a été noté sur chaque cage. La température de la pièce a été maintenue à une température d'environ 23 °C ± 2 °C avec une humidité relative de 60 ± 10 %.

Les animaux ont été nourris une fois par jour (sauf le dimanche) avec des croquettes pour chat du commerce, vendues par la société Harlan - Teklad selon les recommandations du fabricant, et ils ont eu de l'eau potable fraîche à volonté.

### c) Compositions testées

La composition A conforme à l'invention a été comparée au produit Frontline® Spot-on cat contenant 10 % (g/100 ml) de fipronil et un mélange d'excipients. Il a été utilisé tel que fourni par le fabriquant.

### d) Traitements

Groupe 1 : Traitement avec la composition A à raison de 0,5 ml par chat, soit 50 mg de fipronil par animal,
Groupe 2 : Traitement avec le produit Frontline® Spot-on cat à raison de 0,5 ml par chat, soit 50 mg de fipronil par animal,
Groupe 3 : Contrôle négatif : pas de traitement.

Le traitement a été appliqué de façon topique, entre les omoplates des chats, en une seule fois au commencement de l'étude (J = 0).

### e) Infestations par les puces / Mesure de l'efficacité des traitements

9 jours avant le commencement de l'étude (J = -9), tous les chats ont été infestés par environ 50 puces de laboratoire, souche *Ctenocephalis felis*, de sexe male ou femelle. Les puces ont ensuite été comptées avant le début du traitement de façon à vérifier la capacité de chacun des chats à héberger des puces. Pour réaliser un comptage, toutes les puces présentes sur un animal sont récoltées par peignage du chat puis comptées après peignage. Le nombre de puces est ainsi déterminé. Après comptage, les puces sont détruites et un nouveau lot d'environ 50 puces est remis sur l'animal le jour précédent l'administration du traitement (J = -1).

La première infestation a eu lieu la veille du traitement (J = -1).

Les ré-infestations ont ensuite eu lieu à J = 7, J = 14, J = 21, J = 28, J = 35, J = 42 et J = 49).

Les puces ont été comptées 48 heures après l'administration du traitement puis 48 heures après chacune des réinfestation (J = 9, J = 16, J = 23, J = 30, J = 37, J = 44 et J = 51).

Pour ce faire, toutes les puces présentes sur un animal sont récoltées par peignage du chat puis comptées après peignage. Le nombre de puces vivantes est ainsi déterminé. Après chaque comptage, 50 nouvelles puces vivantes sont remises sur le chat.

A chaque comptage, l'efficacité du traitement a été calculée selon l'équation suivante :

$$\% \text{ d'efficacité} = 100 \text{ x } (NP_vC - NP_vT) / NP_vC$$

dans laquelle :

- $NP_vC$ est la moyenne géométrique du nombre de puces vivantes comptées sur les chats du groupe 3 (contrôle) ;
- $NP_vT$ est la moyenne géométrique du nombre de puces vivantes comptées sur les chats d'un groupe ayant reçu un traitement (Groupes 1 ou 2).

Un traitement est dit efficace si le pourcentage d'efficacité est supérieur ou égal à 95%.

**2) Résultats**

Les résultats obtenus sont représentés dans le tableau III ci-après qui représente, pour chaque groupe, le pourcentage de puces mortes (moyennes géométriques) comptabilisées à différents temps après l'infestation :

**TABLEAU III**

| Jours | GROUPE 1 (Composition A) | GROUPE 2 (Frontline ® Spot-on cat) |
|---|---|---|
| J = 2 | 95.1 | 99.4 |
| J = 9 | 100.0 | 100.0 |
| J = 16 | 99.2 | 100.0 |
| J = 23 | 100.0 | 100.0 |
| J = 30 | 99.7 | 100.0 |
| J = 37 | 99.7 | 96.0 |
| J = 44 | **96.3** | **74.3** |
| J = 51 | **95.6** | **64.1** |

Un suivi de la tolérance locale a été réalisé à 1 heures, 6 heures, 24 heures et 48 heures après l'application du traitement. Lors de ces observations, quelques changements cosmétiques au site d'application de la composition A ont été notés, dont en particulier des phénomènes de méchage (poils collés avec formation d'épis) et un aspect gras à 1 heures et 6 heures après application. Quelques dépôts blancs (poudre blanche, cristaux) à l'extrémité des poils ont par ailleurs été observés à 24 heures et à 48 heures après l'application du traitement. Néanmoins, aucun signe d'intolérance locale ou générale n'a été rapporté pour la composition A tout au long de l'essai.

Ces résultats démontrent que :

- la composition A conforme à la présente invention reste efficace (détermination au bout de 48 heures après l'infestation) pendant 7 semaines (J = 51) contre les infestations par les puces chez le chat ;
- le produit Frontline ® Spot-on cat ne reste efficace (détermination au bout de 48 heures après l'infestation) que pendant 5 semaines (J = 37) contre les infestations par les puces chez le chat.

L'efficacité et la meilleure rémanence de la composition A conforme à la présente Invention sont ainsi clairement démontrées.

**Revendications**

1. Composition pharmaceutique liquide, **caractérisée par le fait qu'**elle renferme :

    - à titre de principe actif, le 5-amino-1-[2,6-dichloro-4-(trifluorométhyl)phényl]-4-(trifluorométhylsulfinyl)-1H-pyrazole-3-carbonitrile (fipronil),
    - au moins 5 % (poids/volume) d'alcool benzylique, et
    - au moins 50 % (poids/volume) d'un solvant organique choisi parmi le monométhyléther du propylèneglycol, le n-butyléther du dipropylèneglycol, le monométhyléther de l'éthylèneglycol, le monoéthyléther de l'éthylèneglycol, le monoéthyléther du diéthylène glycol, le propylène glycol, et leurs mélanges,

étant entendu que ladite composition est exempte d'alcool en $C_1$-$C_4$,
ladite composition étant **caractérisée en ce qu'**elle est conditionnée en pipettes monodoses.

2. Composition selon la revendication 1, **caractérisée par le fait que** le fipronil représente de 5 à 15 % (poids/volume).

3. Composition selon l'une quelconque des revendications précédentes, **caractérisée par le fait que** l'alcool benzylique représente de 25 à 35 % (poids/volume).

4. Composition selon l'une quelconque des revendications précédentes, **caractérisée par le fait que** le solvant organique est le monoéthyléther du diéthylèneglycol.

5. Composition selon l'une quelconque des revendications précédentes, **caractérisée par le fait qu'**elle renferme en outre un ou plusieurs antioxydants choisis parmi le butylhydroxyanisole, le butylhydroxytoluène, le gallate de propyle, le palmitate d'ascorbyle, les extraits de romarin et leurs mélanges.

6. Composition selon l'une quelconque des revendications précédentes, **caractérisée par le fait qu'**elle comprend un ou plusieurs principes actifs antiparasitaires additionnels.

7. Composition selon la revendication 6, **caractérisée par le fait que** les principes actifs antiparasitaires additionnels sont choisis parmi les acaricides, les inhibiteurs du développement des puces et des tiques, les endoparasiticides et les principes actifs contre les phlébotomes et les ectoparasites des animaux domestiques.

8. Composition selon l'une quelconque des revendications précédentes, **caractérisée en ce que** les pipettes comprennent entre 0,3 et 1,5 ml de ladite composition.

9. Composition pharmaceutique liquide telle que décrite à l'une quelconque des revendications précédentes, pour son utilisation par application topique pour la prévention et/ou le traitement des infestations par les puces chez les animaux domestiques.

10. Composition selon l'une l'une quelconque des revendications 1 à 8 pour son utilisation selon la revendication 9, **caractérisée par le fait que** ladite composition est destinée à un chat et est administrée en une quantité variant de 0,3 à 1,5 ml.

11. Composition selon l'une l'une quelconque des revendications 1 à 8 pour son utilisation selon la revendication 9, **caractérisée par le fait que** ladite composition est destinée à un chien et est administrée en une quantité variant de 0,3 à 6 ml.

12. Procédé de préparation d'une composition selon l'une quelconque des revendications 1 à 8, **caractérisé en ce qu'**il comprend une étape de dilution du fipronil et éventuellement du ou des principes actifs antiparasitaires additionnels dans l'alcool benzylique et le ou les solvants organiques utilisés.

13. Utilisation d'un véhicule consistant en :

    - au moins 5 % (poids/volume) d'alcool benzylique, et
    - au moins 50 % (poids/volume) d'un solvant organique choisi parmi le monométhyléther du propylèneglycol, le n-butyléther du dipropylèneglycol, le monométhyléther de l'éthylèneglycol, le monoéthyléther de l'éthylèneglycol, le monoéthyléther du diéthylène glycol, le propylène glycol, et leurs mélanges,

    pour préparer une composition pharmaceutique liquide à base de 5-amino-1-[2,6-dichloro-4-(trifluorométhyl)phényl]-4-(trifluorométhylsulfinyl)-1H-pyrazole-3-carbonitrile (fipronil) plus rémanente dans la prévention et le traitement des infestations par les puces chez les animaux domestiques.

14. Utilisation selon la revendication 13, **caractérisée en ce que** le solvant organique est le monométhyléther du diéthylèneglycol.

**15.** Utilisation selon la revendication 13 ou la revendication 14, **caractérisée en ce que** l'alcool benzylique représente de 25 à 35 % (poids/volume).

**Patentansprüche**

**1.** Flüssige pharmazeutische Zusammensetzung, **dadurch gekennzeichnet, dass** sie umfasst:

- als aktiven Inhaltsstoff 5-Amino-1-[2,6-dichlor-4-(trifluormethyl)phenyl]-4-(trifluormethylsulfinyl)-1H-pyrazol-3-carbonitril (Fipronil),
- mindestens 5 % (Gewicht/Volumen) Benzylalkohol, und
- mindestens 50 % (Gewicht/Volumen) eines organischen Lösungsmittels, ausgewählt aus dem Monomethylether von Propylenglykol, dem n-Butylether von Dipropylenglykol, dem Monomethylether von Ethylenglykol, dem Monoethylether von Ethylenglykol, dem Monoethylether von Diethylenglykol, dem Propylenglykol und Mischungen davon,

wobei die Zusammensetzung ohne $C_1$-$C_4$-Alkohol ist,
wobei die Zusammensetzung **dadurch gekennzeichnet ist, dass** sie in Eindosen-Pipetten verpackt ist.

**2.** Zusammensetzung gemäß Anspruch 1, **dadurch gekennzeichnet, dass** das Fipronil von 5 bis 15 % (Gewicht/Volumen) ausmacht.

**3.** Zusammensetzung gemäß einem der vorhergehenden Ansprüche, **dadurch gekennzeichnet, dass** der Benzylalkohol von 25 bis 35 % (Gewicht/Volumen) ausmacht.

**4.** Zusammensetzung gemäß einem der vorhergehenden Ansprüche, **dadurch gekennzeichnet, dass** das organische Lösungsmittel Diethylenglykolmonoethylether ist.

**5.** Zusammensetzung gemäß einem der vorhergehenden Ansprüche, **dadurch gekennzeichnet, dass** sie ferner ein oder mehrere Antioxidantien umfasst, die aus dem Butylhydroxyanisol, dem Butylhydroxyltoluol, dem Propylgallat, dem Ascorbylpalmitat, den Rosmarinextrakten und Mischungen davon ausgewählt sind.

**6.** Zusammensetzung gemäß einem der vorhergehenden Ansprüche, **dadurch gekennzeichnet, dass** sie einen oder mehrere zusätzliche aktive Inhaltsstoffe gegen Parasiten umfasst.

**7.** Zusammensetzung nach Anspruch 6, **dadurch gekennzeichnet, dass** die zusätzlichen aktiven Inhaltsstoffe gegen Parasiten aus den Insektiziden, den Inhibitoren der Entwicklung von Flöhen und Zecken, den Insektiziden gegen Endoparasiten und den aktiven Inhaltsstoffen gegen die Phlebotome und gegen die Ektoparasiten von Haustieren ausgewählt sind.

**8.** Zusammensetzung gemäß einem der vorhergehenden Ansprüche, **dadurch gekennzeichnet, dass** die Pipetten zwischen 0,3 und 1,5 ml der Zusammensetzung enthalten.

**9.** Flüssige pharmazeutische Zusammensetzung gemäß einem der vorhergehenden Ansprüche für ihre Verwendung topischen Anwendung zur Vorbeugung und/oder zur Behandlung des Befalls durch Flöhe bei Haustieren.

**10.** Zusammensetzung gemäß einem der Ansprüche 1 bis 8 für ihre Verwendung gemäß Anspruch 9, **dadurch gekennzeichnet, dass** die Zusammensetzung für eine Katze bestimmt ist und in einer Menge im Bereich von 0,3 bis 1,5 ml verabreicht wird.

**11.** Zusammensetzung gemäß einem der Ansprüche 1 bis 8 für ihre Verwendung gemäß Anspruch 9, **dadurch gekennzeichnet, dass** die Zusammensetzung für eine Katze bestimmt ist und in einer Menge im Bereich von 0,3 bis 6 ml verabreicht wird.

**12.** Verfahren für die Herstellung einer Zusammensetzung gemäß einem der Ansprüche 1 bis 8, **dadurch gekennzeichnet, dass** es einen Schritt der Verdünnung des Fipronils und eventuell des oder der zusätzlichen antiparasitären aktiven Inhaltsstoffe in dem Benzylalkohol und in dem oder den verwendeten organischen Lösungsmitteln umfasst.

13. Verwendung eines Vehikels, das besteht aus:

- mindestens 5 % (Gewicht/Volumen) Benzylalkohol, und
- mindestens 50 % (Gewicht/Volumen) eines organischen Lösungsmittels, ausgewählt aus dem Monomethylether von Propylenglykol, dem n-Butylether von Dipropylenglykol, dem Monomethylether von Ethylenglykol, dem Monoethylether von Ethylenglykol, dem Monoethylether von Diethylenglykol, dem Propylenglykol und Mischungen davon,

für die Herstellung einer flüssigen pharmazeutischen Zusammensetzung dass es 5-Amino-1-[2,6-dichlor-4-(trifluormethyl)phenyl]-4-(trifluormethylsulfinyl)-1H-pyrazol-3-carbonitril (Fipronil) umfasst und dass einer remanenter Verwendung topischen Anwendung zur Vorbeugung und/oder zur Behandlung des Befalls durch Flöhe bei Haustieren induziert.

14. Verwendung nach Anspruch 13, dass das organische Lösungsmittel das Monoethylether von Diethylenglykol ist.

15. Verwendung nach Anspruch 13 oder Anspruch 14, **dadurch gekennzeichnet, dass** der Benzylalkohol von 25 bis 35 % (Gewicht/Volumen) darstellt.

**Claims**

1. A liquid pharmaceutical composition, **characterized in that** it contains:

   - as active principle, 5-amino-1-[2,6-dichloro-4-(trifluoromethyl)phenyl]-4-(trifluoromethylsulfinyl)-1H-pyrazole-3-carbonitrile (fipronil),
   - at least 5% (weight/volume) of benzyl alcohol, and
   - at least 50% (weight/volume) of an organic solvent chosen from propylene glycol monomethyl ether, dipropylene glycol n-butyl ether, ethylene glycol monomethyl ether, ethylene glycol monoethyl ether, diethylene glycol monoethyl ether and propylene glycol, and mixtures thereof,

   it being understood that said composition is free of $C_1$-$C_4$ alcohol,
   **characterized in that** it is conditioned in single-dose pipettes.

2. The composition as claimed in claim 1, **characterized in that** the fipronil represents from 5% to 15% (weight/volume).

3. The composition as claimed in any one of the preceding claims, **characterized in that** the benzyl alcohol represents from 25% to 35% (weight/volume).

4. The composition as claimed in any one of the preceding claims, **characterized in that** the organic solvent is diethylene glycol monoethyl ether.

5. The composition as claimed in any one of the preceding claims, **characterized in that** it also contains one or more antioxidants chosen from butylhydroxyanisole, butylhydroxytoluene, propyl gallate, ascorbyl palmitate and rosemary extracts, and mixtures thereof.

6. The composition as claimed in any one of the preceding claims, **characterized in that** it comprises one or more additional antiparasitic active principles.

7. The composition as claimed in claim 6, **characterized in that** the additional antiparasitic active principles are chosen from acaricides, flea and tick growth inhibitors, endoparasiticides and active principles against pet ectoparasites and sand flies.

8. The composition as claimed in any one of the preceding claims, **characterized in that** said pipettes comprise an amount of said composition ranging from 0.3 to 1.5 ml.

9. The composition according to any one of the preceding claims for its topical use for the prevention and/or treatment of pets flea infestations.

**10.** The composition according to any one of claims 1 to 8 for its use according to claim 9, **characterized in that** said composition is intended to be administered in an amount ranging from 0.3 to 1.5 ml to cats.

**11.** The composition according to any one of claims 1 to 8 for its use according to claim 9, **characterized in that** said composition is intended to be administered in an amount ranging from 0.3 to 6 ml to dogs.

**12.** Process of preparation of a composition according to anyone of claims 1 to 8, **characterized in that** it comprises a step of dilution of fipronil and optionally of the additional antiparasitic active principle(s) in benzyl alcohol and the organic solvent(s) used.

**13.** The use of a vehicle consisting of:

- at least 5% (weight/volume) of benzyl alcohol, and
- at least 50% (weight/volume) of an organic solvent chosen from propylene glycol monomethyl ether, dipropylene glycol n-butyl ether, ethylene glycol monomethyl ether, ethylene glycol monoethyl ether, diethylene glycol monoethyl ether and propylene glycol, and mixtures thereof,

for the preparation of a liquid pharmaceutical composition comprising 5-amino-1-[2,6-dichloro-4-(trifluoromethyl)phenyl]-4-(trifluoromethylsulfinyl)-1H-pyrazole-3-carbonitrile (fipronil), said liquid composition being more remanent for the prevention and the treatment of pet flea infestations.

**14.** The use as claimed in claim 13, **characterized in that** the organic solvent is diethylene glycol monoethyl ether.

**15.** The use as claimed in claim 13 or 14, **characterized in that** the benzyl alcohol represents from 25% to 35% (weight/volume).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0295117 A
- EP 0352944 A
- EP 0881881 A